# EUROPEAN PATENT SPECIFICATION

(11) **EP 2 493 420 B1**
(45) Date of publication and mention of the grant of the patent: **22.07.2015**
(21) Application number: 10773693.6
(22) Date of filing: 29.10.2010
(51) Int. Cl.: A61F 2/08, A61F 2/00, D03D 1/00, D03D 3/06, A61B 17/06

(54) **KNOT SLIP RESISTANT WOVEN CORD**
KNOTENRUTSCHBESTÄNDIGE WEBSCHNUR
CORDON À NOEUDS TISSÉ RÉSISTANT AU GLISSEMENT

(30) Priority: 29.10.2009 GB 0918919
(43) Date of publication of application: 05.09.2012
(73) Proprietor: Xiros Limited, Leeds LS19 7UE (GB)
(72) Inventor: LORRISON, Jonathan, West Yorkshire (GB); TIDBALL, Lauren, Elizabeth, West Yorkshire LS19 7UE (GB); BEEVERS, David, Leeds LS19 7UE (GB)
(74) Representative: Orr, Robert
(86) International application number: PCT/GB2010/051817
(87) International publication number: WO 2011/058340

(56) References cited:
- EP-A1- 0 916 312
- WO-A2-2008/116127
- WO-A2-2009/109778
- WO-A2-2010/043868
- US-A- 4 640 317
- US-A1- 2008 161 837

## Description

The present invention relates to a woven cord comprising interwoven wefts and warps having different weave patterns extending over regions of the cord to inhibit slip of the cord when knotted.

Soft tissue (tendon and ligament) ruptures are common amongst sports professionals and the elderly for example. A variety of different surgical procedures have been developed to repair soft tissue tears based on either biological graft tissue or an implantable prosthetic exhibiting physical and mechanical properties that match or exceed those of the damaged soft tissue.

GB 2151487 discloses a prosthetic filament based cord for use in the replacement of damaged ligaments and tendons. Carbon fibres are held together in a parallel array to form a core surrounded by a braided sheath. Tissue ingrowth at the prosthetic is facilitated by braiding the sheath with an open structure. The ends of the cord taper to form tails for ease of looping and securing to bone screws and the like.

US 4,728,329 discloses a prosthetic tape formed of concentric flexible sleeve-like elements formed of braided filaments. The cord is used as a transverse ligament for a knee joint in which a flexible zone is provided at a central region which is of greater width than the end regions.

US 5,217,495 discloses a semi bioabsorbable prosthetic for replacement of the human anterior cruciate ligament. The cord is constructed from a composite yarn having a non-bioabsorbable core surround by a bioabsorbable or semi-bioabsorbable sheath yarn.

US 2004/0078089 discloses a textile prosthetic constructed from firstly binding yarns positioned at anchorage regions of the cord for attachment to fixation screws and the like and secondly tensile load bearing filaments to inhibit resistance to stretch when tensile load is applied in one or more directions.

US 2005/0192631 discloses a suture tape constructed from a braided high strength surgical material. A tubular suture extends along the length of the tape to provide a backbone to the construct extending through a flat braided middle portion. Transition sections at each end of the central braided part taper to allow the suture tape to pass through apertures during surgical procedures.

GB 2458878 discloses an implantable prosthetic cord having a main length of a first width and end portions of reduced width relative to the main length. The cord is particularly suitable to attach ruptured soft tissue, such as ligaments and tendons, to bone anchor sites in turn providing a prosthetic scaffold to facilitate tissue ingrowth.

WO 2009/109778 discloses a medical cord and surgical procedure for the connection of a first tissue site to a second tissue site. The cord comprises a main length having a width and end portions having respective widths being less than the width of the main length. This provides a medical cord that can be manipulated during surgical procedures in which the cord may be required to be threaded through apertures and tunnels. The cord can be used to attach soft tissue, including ligaments and tendons to bone anchor sites and provides a prosthetic scaffold to facilitate tissue ingrowth at the repair site.

In general, secure knotting of the prosthetic tapes, cords and sutures is of particular importance so that the implanted devices do not slip when initially anchored in position. When securely knotted in position, tissue repair can then proceed effectively via cell ingrowth at the prosthetic such that ultimately biological fixation supercedes the initial anchorage.

Knot slip has been identified as a particular problem with existing prosthetic cords. In particular, appreciable slip may be observed post surgical procedure which is significantly disadvantageous for the patient. Firstly, the implanted ligament length is increased which provides an unstable joint if allowed to heal. Secondly, knot slippage is destructive to initial stage biological ingrowth.

There is therefore a need for an implantable reconstructive device that both matches or exceeds the performance of the soft tissue to which it is attached or is to replace whilst enabling secure first stage fixation by avoidance of knot slip.

Accordingly, the inventors provide a woven prosthetic device optimised for both performance as a replacement for a tendon or ligament and to inhibit knot slip. The cord length comprises an alternating width profile which, when knotted, provides an anchorage that is resistant to slip in response to tensile loading forces. The alternating width profile provides ridges and troughs extending transverse to the longitudinal axis of the cord that significantly increase the frictional resistance at the region of the knot and prevent the yarns slipping when placed under tension.

The alternating width profile is created by interweaving the cord warps and wefts according to a plurality of different weave patterns along the length of the cord. In particular, a first weave pattern may be of greater width than a neighbouring second weave pattern such that by positioning the first and second sections side-by-side according to an alternating arrangement, the cord width alternates from relative thick to thin.

According to a first aspect of the present invention there is provided a woven surgical cord as claimed in claim 1.

Variation in the width profile of the cord and in particular the difference in the width of the cord between the thick and thin section is created by variation of the weave pattern at the different sections of cord.

In particular, and preferably the spacing between the wefts in the first section is greater than the spacing between the wefts in the second section.

Preferably, the cord comprises a greater number of wefts in the second section than first section.

Optionally, the number of wefts in the second section is substantially 40% to 60% more than the wefts in the first section.

Preferably, a relative width of the cord at the second section is 30% to 60% of the width of the cord at the first section.

Optionally, the spacing between warps in the first section is greater than the spacing between warps in the second section.

Preferably, the spacing between the warps in the first section is 40% to 60% more than the spacing of the warps in the second section.

Preferably, a reduction in the width of the cord is provided as the wefts are looped around at least some of the warps in the second section.

Optionally, the thickness of the cord tapers between the first and second sections according to a linear or curved profile.

Optionally, the cord may comprise a single yarn or a plurality of yarns to form a composite cord. The yarns may vary in thickness and/or material. Optionally, the yarns comprise polyester.

The alternating width profile may extend substantially the full length of the cord. Alternatively, the alternating width profile may extend over the end regions of the cord being separated by a region that is devoid of the alternating width profile and of substantially uniform width. In particular, the cord length between the profiled end regions may comprise a substantially planar, flat, tape-like profile.

According to a second aspect of the present invention there is provided a method of manufacturing a woven surgical cord as claimed in claim 13.

Preferably, the method comprises interweaving the wefts and warps with variation in the spacing between the wefts and/or warps at the first and second sections.

Preferably, the wefts and warps are interwoven so that the number of warps and/or wefts is different at the first and second sections.

Preferably, the method comprises interweaving wefts in the first section under a different tension relative to the wefts in the second section.

In particular, the respective tension in the wefts, during manufacture, is greater in the second section that the first section.

Preferably, the method comprises interweaving warps in the first section under different tension relative to the tension of the warps in the second section.

Preferably, the tension in the warps, during manufacture, is greater than in the first section than the second section.

Preferably, the cord is manufactured in a shuttle loom.

Preferably, the cord comprises warps ending the full length from one end to the other. Importantly, the warps extend from any central woven section of the outermost ends of the end sections comprising the undulating width profile. These warps are configured to be load bearing and to withstand tensile forces imposed upon the cord in response to joint movement.

A method of surgical repair is also disclosed, which does not fall within the scope of the invention as claimed. In one example, the method of surgical repair comprises: securing a cord to a first biological tissue site involving tying said cord to form a knot, the cord comprising: interwoven warps and wefts extending over a length of the cord; a first weave pattern created by the warps and wefts extending over a first section of the length of cord; a second weave pattern created by the warps and wefts extending over a second section of length of the cord, the second section having a cord width less than a cord width of the first section; wherein the first and second sections repeat according to an alternating arrangement over a region along the length of the cord;
wherein the arrangement of the first and second sections provide an alternating width profile along the region of cord; securing said cord to a second biological tissue site involving tying said cord to form a second knot; wherein said cord extends between the first and second tissue sites.

Preferably, the first tissue site comprises flexible tissue and said second tissue site comprises bone.

Preferably, the method further comprises: forming at least one hole at said first tissue site; and threading said cord through said at least one hole to form a loop in said cord.

Preferably, the method further comprising: drilling at least one through bore at said second tissue site; threading said cord through said at least one through bore; and securing said cord in position within said at least one through bore to prevent said cord from being retracted from said through bore. Preferably, the first tissue site comprises rotator cuff tissue and said second tissue site comprises bone.

Preferably, second tissue site comprises a humeral head wherein said step of securing said cord to said humeral head comprises drilling two through bores through a region of said humeral head and threading end regions of said cord through said through bores and knotting said cord ends together once threaded through said through bores.

Within the specification the term 'pattern' refers to the way in which the warps and wefts are interwoven. Parameters that may be varied to create a different weave pattern include the relative spacing between the wefts and/or warps; the density of the wefts and/or warps; the tension during weaving in the wefts and/or warps; the relative alignment by which the warps and wefts sit relative to one another.

The term 'width' within the specification refers to the distance across the cord perpendicular to the cord longitudinal axis. This term includes a distance across a cord having any polygonal cross section including a circular, square, rectangular, or elliptical shape. Additionally, the width variation may be uniform or non-uniform along the region of the cord.

A specific implementation of the present invention will now be described, by way of example only, and with reference to the accompanying drawings in which:
Figure 1a is a cross sectional side elevation view through a shoulder joint of a human having a torn rotary cuff tissue reattached to the humeral head by a prosthetic according to a specific implementation of the present invention;
Figure 1b illustrates a perspective view of the repaired shoulder joint of Figure 1a;
Figure 2a illustrates schematically a plan view of a cord comprising an alternating width profile according to a specific implementation of the present invention;
Figure 2b illustrates a further specific implementation of the alternating width profile of the cord of Figure 2a;
Figure 2c illustrates a further specific implementation of the alternating width profile of the cord of Figure 2a;
Figure 2d illustrates a further specific implementation of the alternating width profile of the cord of Figure 2a;
Figure 3a is a cross sectional view through A-A of the cord of Figure 2a;
Figure 3b is a side view of the section of cord at A-A of figure 2a;
Figure 3c is a cross sectional view through B-B of the cord of Figure 2a;
Figure 4 illustrates schematically a plan view of a cord comprising an alternating width profile at end regions either side of a central non-profiled region according to a specific implementation of the present invention;
Figure 5 illustrates a cord comprising an alternating width profile extending substantially its full length according to a specific implementation of the present invention;
Figure 6a to 6k illustrates schematically a plan view of a cord comprising an alternating width profile at end regions either side of a central region according to further specific implementations of the present invention;
Figure 7 is a graph of knot slip test results for a prior art cord expressed as load versus extension;
Figure 8 is a graph illustrating the mean load to achieve knot slip for a cord of the subject invention according to two different knot types in two environments - dry and wet to simulate biological fluids.

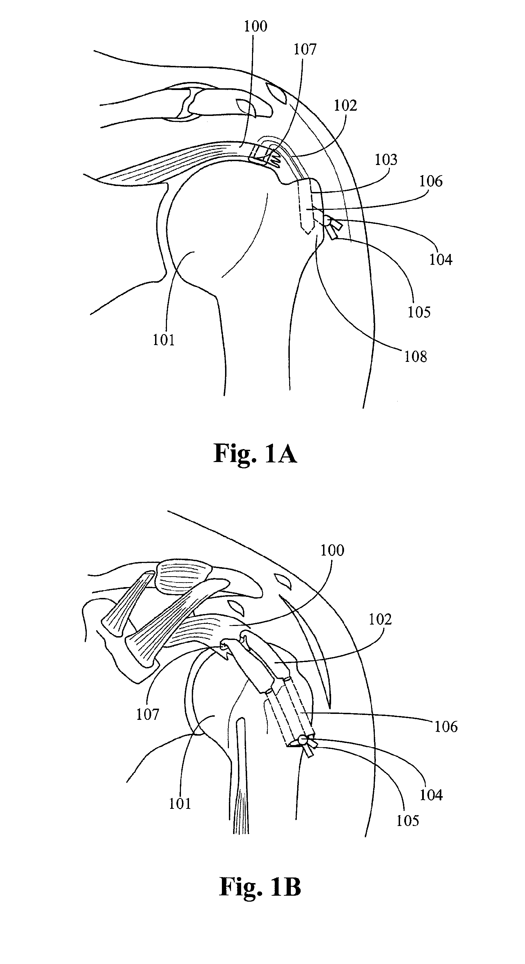

Referring to Figures 1a and 1b, the present prosthetic cord finds particular application in the repair of damaged or torn soft tissue ligaments and tendons. An example application is the repair of a torn rotator cuff tissue 100 located at a human shoulder. A typical injury of the rotator cuff involves separation of the cuff tissue 100 from the humerus and in particular the humeral head 101. The surgical cord comprises a central section 102 bordered at each end by end regions 106. The central section 102 is threaded through the torn end region 107 of rotator cuff 100. The flat, tape-like central section 102 extends from the rotator cuff 100 to sit over a region of the humeral head 101.

End regions 106 are of narrower width than central section 102 and are configured to be easily threaded through suitable bone tunnels 103 formed within the humerus 101. The two ends 106 are threaded respectively through two bone tunnels 103 extending into the greater tuberosity 108. The very end portions 105 at each tape end 106, emerging from the bone tunnels 103, are then tied together in a knot on the outside of the humerus 101 whilst maintaining the tension in the rotator cuff tissue 100.

Advantageously, each end section 106 comprises a ribbed profile extending longitudinally between central region 102 and the cord ends 105. This ribbed profile prevents slip of the knot 104 when subjected to tensile loading forces encountered by post surgery manipulation of the shoulder joint during rehabilitation and prior to second stage biological fixation.

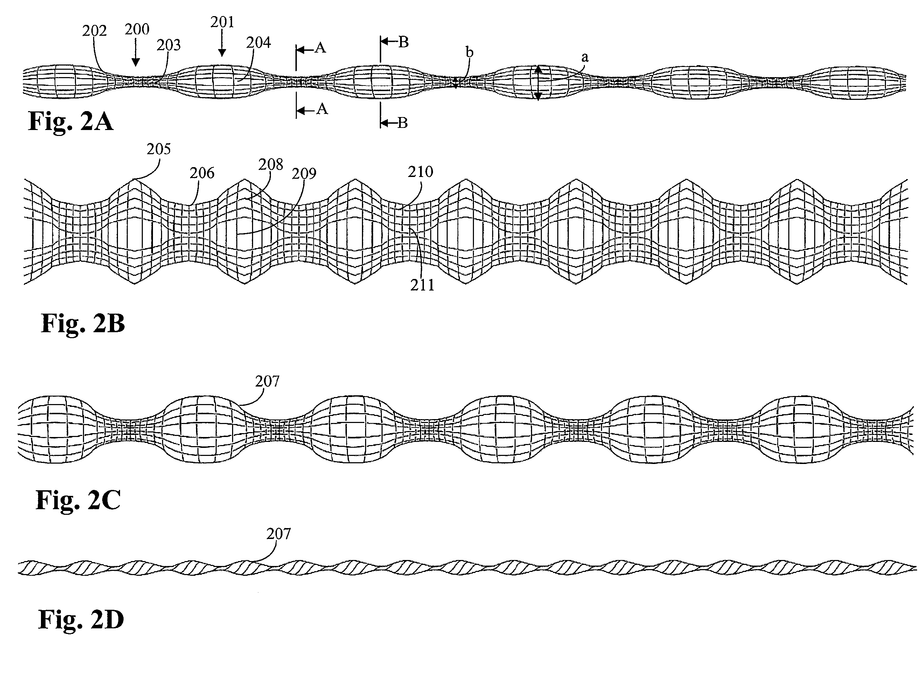

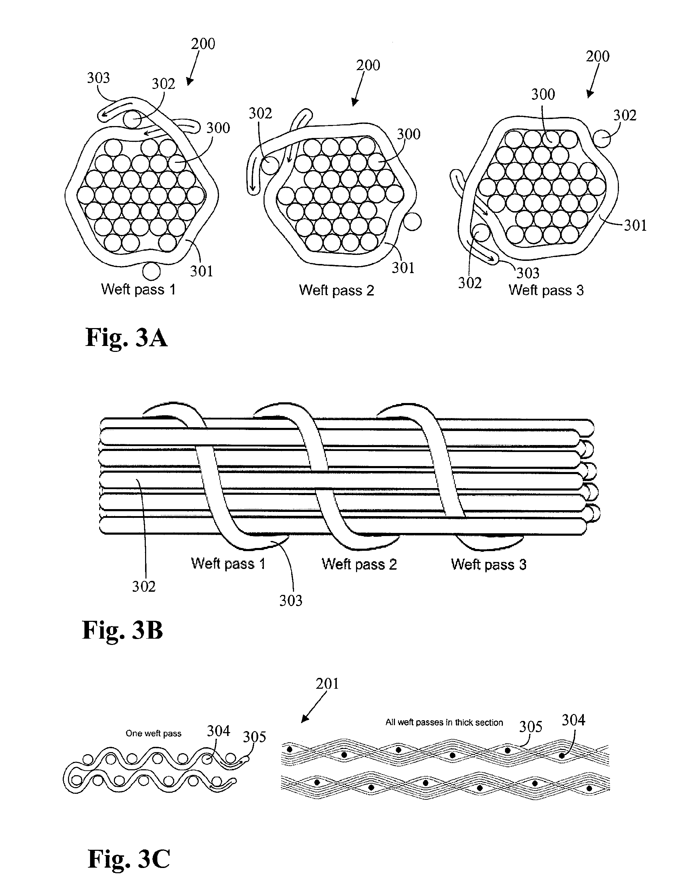

Figures 2a to 2d illustrate sections of the present prosthetic cord over end regions 106. The ribbed profile is created by variation in the weave pattern along the cord length. A first section 201 comprises a first weave pattern 204 whilst a second section 200 comprises a second weave pattern 203 such that the width of the cord 'a' at the first section 201 is greater than the width of the cord 'b' at the second section 200. The difference in the thickness of the cord at the two sections 201, 200 is created, in part, by the change in the weave pattern illustrated further with reference to Figures 3a and 3b.

The relative spacing between warps 208 in the wider section 201 is greater than the corresponding spacing between the warps 210 in the relatively narrow section 200. Moreover, the spacing between wefts 209 in the wider section 201 is greater than the corresponding spacing between wefts 211 in the narrow section 200. Furthermore, the cord comprises a greater number of wefts 211 in the narrow sections 200 which act to pull-in the warps 210. Accordingly, weave pattern 204 may be regarded as less dense than weave pattern 203.

According to specific implementations, the transition between the wider (201) and narrower (200) sections may follow a smooth or curved profile 202, 207. Alternatively, the wider sections 201 may be formed with relatively sharp peaks 205 that descend steeply into intermediate troughs 206 at the narrow sections 200.

According to specific implementations, the width 'b' of cord at the narrow section 200 is approximately 30% to 60% the width 'a' of the wide section 201. Additionally, the warps in the weave pattern 203 in the narrow sections 200 are approximately 40% to 60% more dense than the arrangement of warps 208 in the weave pattern 204 of the wider section 201. According to a particular weave pattern, there may be approximately 50% more weft 211 in the narrow section 200 than the weft 209 in the wide section 201.

Figures 3 a to 3c illustrate the difference in the weave pattern at the wide section 201 and narrow section 200. So as to constrict the width of cord at section 200, weft 301 is looped around a central bundle of warp 300. A series of outermost warp 302 is then interwoven with weft 303 to increase structural integrity. In contrast, and referring to Figure 3c, at the wider sections 201, the warp 304 and weft 305 are interwoven according a more conventional lattice-like weave pattern.

The present cord is typically manufactured on a shuttle loom using a single yarn. Variation in the tension of the warp and weft 300, 303, during weaving, is an important parameter in the creation of the alternating width profile along the length of the cord. In particular, the warp 304 at wider section 201 is maintained at high tension whilst interlaced with the weft of relative low tension. In contrast, high tension is applied to the weft 301 at the narrow section 201 with a corresponding lower tension on the warps 300. Effectively, the weft 301, at the thin section provide a radially constricting force to the warp 300 whilst the warp 304 at the thicker sections, at relative high tension, are not radially constricted by the relatively low tension interwoven weft 305. The relative low density of the weft 305 in the wider sections 201 is also important so as to minimise the inward directing radial forces exerted upon the warps 304. Once manufactured, the resulting tension of the yarn (warps and weft) is equal. An example of the tension difference between warps 304 and 300, during manufacture may be in the region of 1kg.

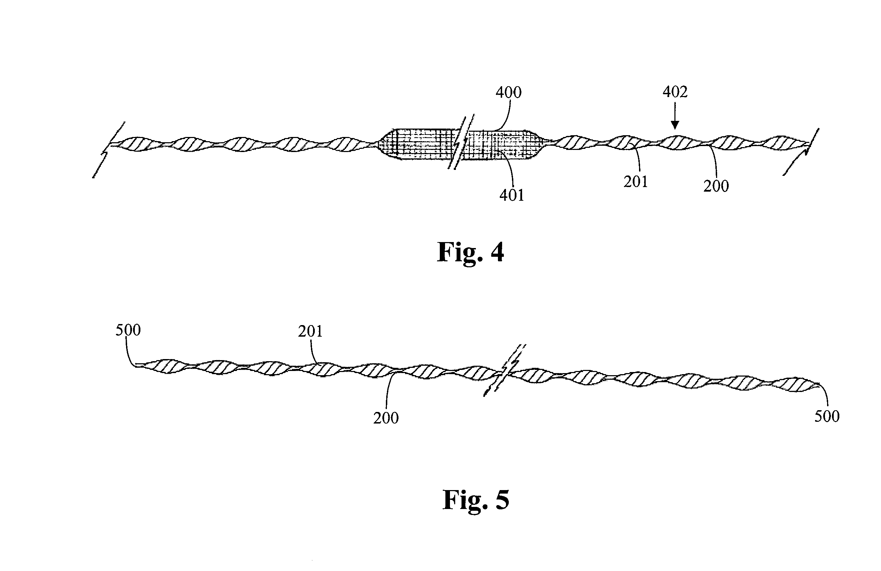

Referring to the embodiment of Figure 4, the prosthetic cord comprises a central woven section 400 having a uniform weave pattern 401 extending across its length. Central section 400 comprises a linear width profile devoid of significant width undulations. Narrower end regions 402 extend from each end of central region 400 and comprise the undulating width profile manifesting as wider sections 201 and narrow sections 200 as described herein. The end sections 402 and central region 400 are formed by the same warps (not shown) that extend the full length of the yarn such that the end sections 402 and central section 400 are formed integrally. These warps (not shown) extending the full length of the woven cord are configured as load bearing yarns to provide a cord capable of withstanding appreciable loading forces when the cord, once implanted, is placed under tensile load by the joint as it is manipulated.

According to Figure 5, the undulations in the width of the cord may extend substantially the full length of the cord between ends 500. The relative thickness difference of the tape between wide sections 201 and narrow sections 200 is uniform between end regions 500. According to further embodiments, this width difference may vary over the length of the cord.

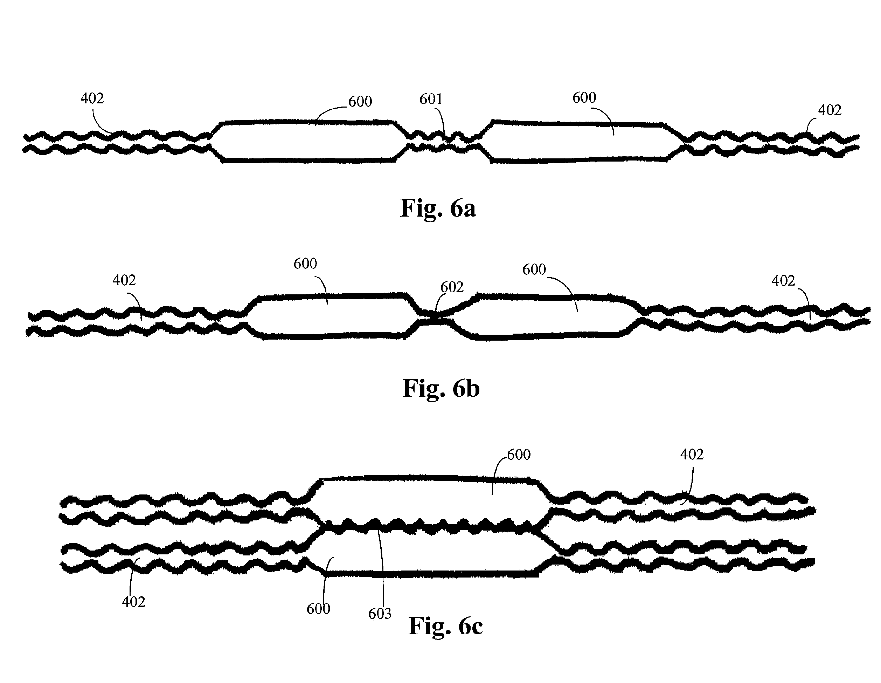

Figures 6a to 6k illustrate further specific implementations of the present invention, in particular, the cord may comprises two substantially planar sections 600 devoid of significant width undulations extending between end regions 402. Referring to figure 6a, an intermediate region 601 extends between the two planar regions 600 and also comprises the undulating width profile present within end sections 402. Alternatively and referring to figure 6b, the central section 602 between tape-like portions 600 may be formed by an extension of the weave and having a much reduced width than regions 600.

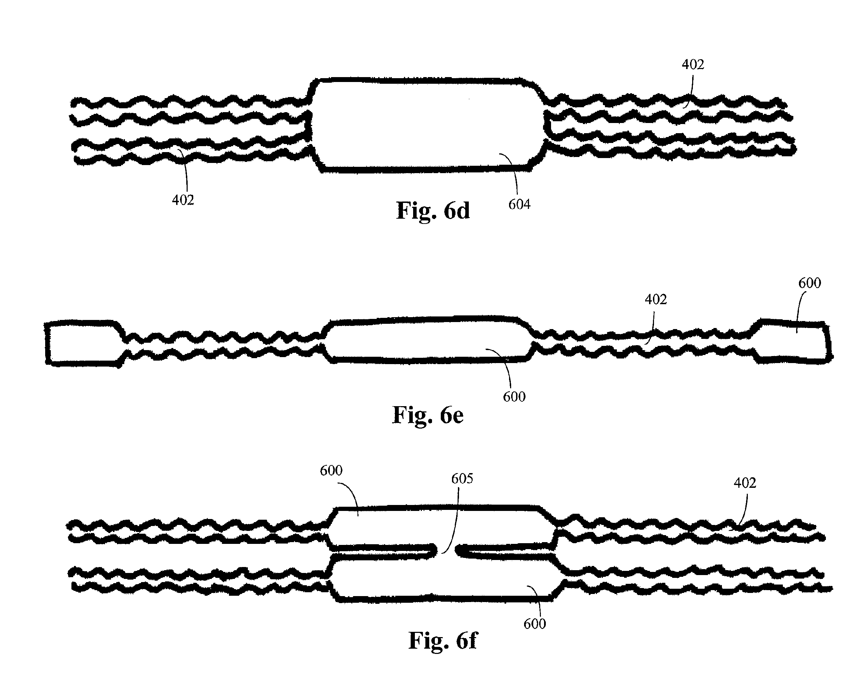

Referring to figure 6c, the cord may comprise two cords illustrated in figure 4 attached together by one of the edges 603 of each central woven section 600. The joining of edges 603 may be provided by separate yarn. Alternatively and referring to figure 6d, two central sections 600 may be interwoven so as to form a integrated central weave section 604 between two end sections 402 that extend from one side and two end sections 402 that extend from a second side. Figure 6e illustrates a further embodiment in which central sections 600 are separated by intermediate sections 402 having the alternating width profile so as to form a cord having a repeating pattern along its length comprising the tape-like sections 600 separated by the narrower profiled regions 402.

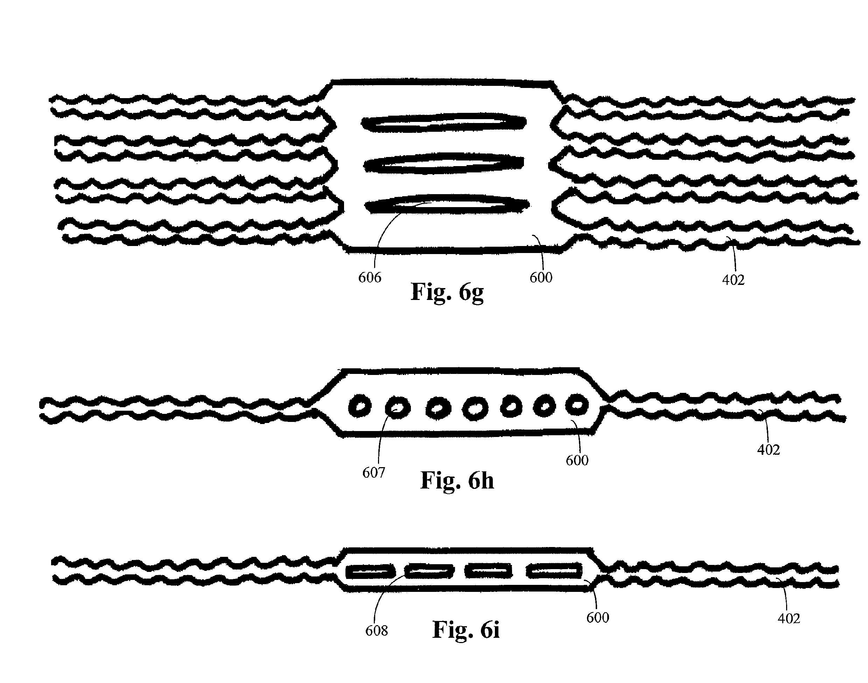

Figure 6f illustrates a further variation on the embodiments of figures 6c and 6d in which the central sections 600 are positioned side-by-side in the same plane and are connected together via a small bridge 605 formed from the same or a different yarn to that of each woven section 600. That is, bridge 605 may be formed integrally or non-integrally with sections 600. Two end regions 402 extend from each respective planar portion 600. Bridge 605 extends approximately mid-way between the regions of central sections 600. Figure 6g illustrates an alternative embodiment in which four central sections 600 are connected together towards their respective ends to form a unitary structure having three apertures 606 extending nearly the full length of each tape-like section 600. The ribbed, undulating end sections 402 extend respectively from both ends of each central section 600 such that a plurality of end sections 402 extend from the unitary central body 600.

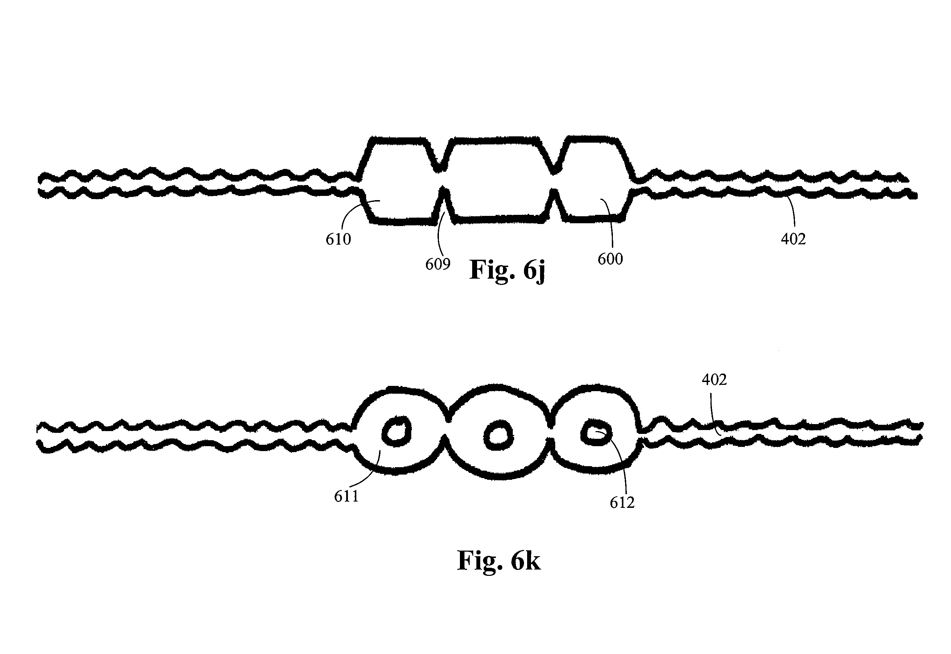

Figure 6h illustrates a further embodiment in which the central section 600 comprises a plurality of circular apertures or holes 607 positioned centrally along the length of section 600 between its ends and from where the end regions 402 extend. Figure 6i illustrates a further embodiment of figure 6h, comprising elongate slots 608 formed within the central woven section 600. In particular, four slots 608 extend the majority of the length of the tape-like structure 600 and are positioned end-to-end. Figure 6j illustrates the planar tape-like structure 600 formed from three segments 610 positioned side-by-side and jointed end-to-end to form a unitary structure. Notches 609 extend from each edge of the woven structure 600 and forms v-shaped indentations. Figure 6k illustrates a further embodiment of the cord of figure 6j in which three annular planar segments 611 are joined together via a small section of their circumference to form a unitary tape-like structure with three circular apertures 612 formed centrally at each segment 611. End sections 402 extend from each end of the central tape-like section illustrated in figures 6h to 6k.

According to further embodiments, the linear width woven sections 600 may be tubular or comprise a hollow central region defined by any cross sectional shape capable of accommodating solid or fluid phase materials. For example, synthetic or biological matter may be accommodated within the structure 600 so as to encourage cell proliferation when implanted in vivo. In particular, the cord may be seeded with cells selected from chondrocytes; fibroblasts; mesenchymal progenitor cells; endosteal cells; periosteal cells; inducible chondroprogenitor cells in extraskeletal organs.

Additionally, the cord may be seeded with a cell culture surface to which cells, in particular, chondrocytic progenitor cells (stem cells) may adhere, proliferate and/or differentiate. Alternatively, the cord may comprise tissue selected from: periosteum, synovium, fascia, retinaculum.

Tests were undertaken to determine the effectiveness of the present width undulations to inhibit knot slip. The acceptance criterion evaluated as part of the investigation was as follows. The knotted cord must be capable of withstanding a mean load at least three standard deviations above 300 Newtons without appreciable slippage. This criterion was investigated with both dry cords and cords coated in 'spin finish' before being knotted over an approximate 200 mm gauge length. 'Spin finishing' was used to simulate the moist *in vivo* environment of the cord. As will be appreciated, the 'spin finish' fluid effectively lubricates the cord and is used specifically as a lubricant during weaving manufacture of the polyester yarns.

The materials used in the present investigation include: fully processed modified poly-tapes (PRF0002); Hounsfield test machine (Model No H5K-S; Serial Number 0945); Large mandrel (C=150mm, D-47.7mm); Large mandrel (C=200mm, D=63.5mm); Small mandrels (D=10mm); Part No. 505-6010 Clamps (WIC 611); Part No. 505-6011 Rubber grips (Michelin A1, 18/23-622, 700x18/23C inner tube); Part No. 505-3121 (or Calibration Reference LAB 25) Calibrated torque wrench. Steel rule; Spin finish (Texturol 1437: GIB 10329).

The cord tested was manufactured from a polyester yarn comprising a 2.5mm diameter at the widest sections and approximately 1.5 mm in the narrow sections. These sections repeat over a 5 mm and 3 mm length, respectively along the cord.

Tests were undertaken with six samples, with each sample knotted according to the following conditions: a dry reef knot 800; a dry triple knot 801; a wet reef knot 802; a wet triple knot 803.

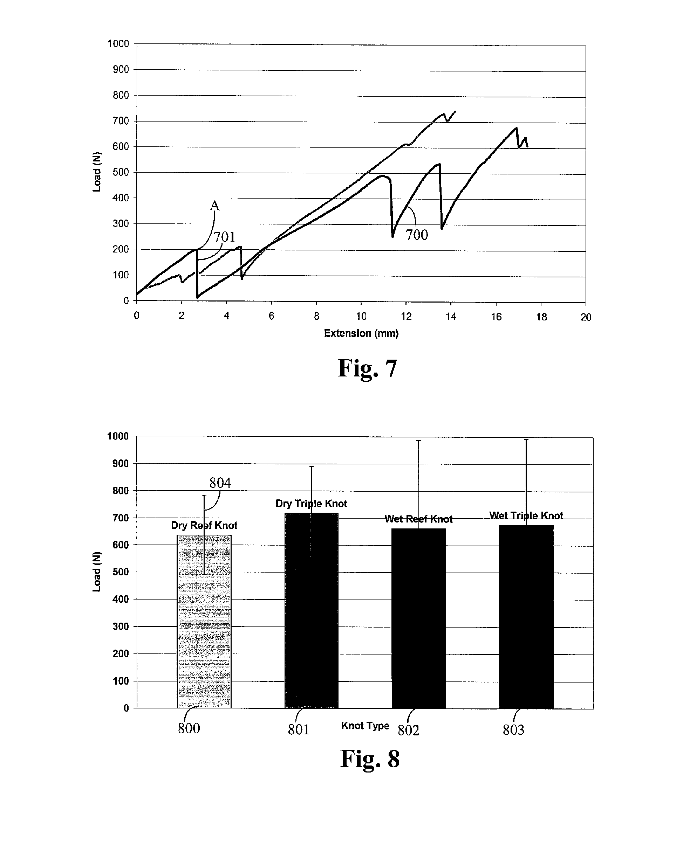

Figure 7 illustrates a prior art prosthetic cord devoid of an undulating width profile at the knotted region. As illustrated, appreciable slip is observed at load and extension point A manifested by the sharp decrease 701 in load over near zero extension. Further slip is then observed at higher loading and extensions 700. Ultimately, conventional, uniform width cord exhibits slipping as approximately 200 Newtons (relatively low load) likely to be encountered during a patient rehabilitation process. This inability to withstand pulling forces of greater than 200 Newtons would lead to the failure of the device as a suitable prosthetic for the repair of a rotator cuff as described with reference to Figures 1a and 1b.

Figure 8 illustrates the results of the knot slip investigation for the different dry and wet knots applied to a cord comprising an undulating width profile according to Figures 2a to 2d and 4 and 5 at the knotted region.

From figure 8 it is evident that the mean value at which significant knot slippage occurred, for all four knot conditions, is at least three standard deviations above 300 Newtons, although there is a greater standard deviation with wetted cords. The present cord therefore passes the acceptance criterion defined above.

## Claims

1. A woven surgical cord comprising:
interwoven warps (208, 210) and wefts (209, 211) extending over a length of the cord;
a first weave pattern (204) created by the warps and wefts extending over a first section (201) of the length of cord;
a second weave pattern (203) created by the warps and wefts extending over a second section (200) of the length of the cord, the second section having a cord width (b) less than a cord width (a) of the first section;
**characterized in that**
a region (106) along a length of the cord comprises a plurality of the first sections and a plurality of the second sections, the first and second sections repeating according to an alternating arrangement over the region of the cord, the arrangement of the first and second sections being such as to provide an alternating width profile formed from ridges (205) and troughs (206) extending transverse to a longitudinal axis of the cord and along the region of the cord.

2. The cord as claimed in claim 1 wherein the spacing between wefts (209) in the first section (201) is greater than the spacing between wefts (211) in the second section (200).

3. The cord as claimed in claims 1 and 2 comprising a greater number of wefts (211) in the second section (200) than the first section (201).

4. The cord as claimed in any preceding claim wherein the spacing between warps (208) in the first section (201) is greater than the spacing between warps (210) in the second section (200).

5. The cord as claimed in any preceding claim wherein the wefts (301) are looped around at least some (300) of the warps in the second section.

6. The cord as claimed in any preceding claim wherein the thickness of the cord tapers between the first and second sections according to a linear or curved profile.

7. The cord as claimed in any preceding claim comprising a flat, substantially planar profile extending over a region (102) of the cord.

8. The cord as claimed in any preceding claim wherein the first and second sections repeat over substantially the full length of the cord.

9. The cord as claimed in any one of claims 1 to 7 wherein the first and second sections extend over end regions (402) of the cord and wherein a region (400) between the end regions is devoid of the first and second sections.

10. The cord as claimed in claim 9 wherein the region between the end regions comprises a substantially uniform width along its length.

11. The cord as claimed in any preceding claim wherein said warps extend over the entire length of said cord.

12. The cord as claimed in any preceding claim wherein the warps extend between end regions (106) and through a central region (102) of the cord.

13. A method of manufacturing a woven surgical cord according to claim 1 comprising:
interweaving warps (208, 210) and wefts (209, 211) to create a first weave pattern (204) extending over a first section (201) of a length of the cord;
interweaving warps and wefts to create a second weave pattern (203) extending over a second section (200) of the length of the cord, the second section having a cord width (b) less than a width (a) of the first section;
interweaving the warps and wefts such that a region (106) along a length of the cord comprises a plurality of the first sections and a plurality of the second sections, the first and second sections repeating according to an alternating arrangement over the region of the cord so that the cord comprises an alternating width profile along the region of its length formed from ridges (205) and troughs (206) extending transverse to a longitudinal axis of the cord.

14. The method as claimed in claim 13 comprising interweaving warps (208) in the first section (201) at greater tension relative to the tension of the warps (210) in the second section (200).

15. The method as claimed in claim 13 comprising interweaving wefts (211) under greater tension in the second section (200) relative to the wefts (209) in the first section (201).

## Patentansprüche

1. Gewebter chirurgischer Faden, umfassend
über die Länge des Fadens reichende verwobene Ketten (208, 210) und Schüsse (209, 211);
ein sich über einen ersten Abschnitt (201) der Länge des Fadens erstreckendes, durch die Ketten und Schüsse gebildetes erstes Webmuster (204);
ein sich über einen zweiten Abschnitt (200) der Länge des Fadens erstreckendes, durch die Ketten und Schüsse gebildetes zweites Webmuster (203), wobei der zweite Abschnitt eine Fadenbreite (b) aufweist, die kleiner als eine Fadenbreite (a) des ersten Abschnitts ist; **dadurch gekennzeichnet,**
**dass** ein Bereich (106) entlang einer Länge des Fadens eine Vielzahl der ersten Abschnitte und eine Vielzahl der zweiten Abschnitte aufweist, wobei die ersten und zweiten Abschnitte sich gemäß einer abwechselnden Anordnung über den Bereich des Fadens wiederholen, wobei die Anordnung der ersten und zweiten Abschnitte derart ist, dass ein abwechselnd durch Verbreiterungen (205) und Einschnürungen (206) geformtes Breitenprofil, welches sich transversal zur Längsachse des Fadens und entlang des Bereichs des Fadens erstreckt, vorgesehen ist.

2. Faden nach Anspruch 1, wobei der Abstand zwischen Schüssen (209) in dem ersten Abschnitt (201) größer ist als der Abstand zwischen Schüssen (211) in dem zweiten Abschnitt (200).

3. Faden nach Anspruch 1 und 2, umfassend eine größere Anzahl Schüsse (211) in dem zweiten Abschnitt (200) als in dem ersten Abschnitt (201).

4. Faden nach einem der vorhergehenden Ansprüche, wobei der Abstand zwischen Ketten (208) in dem ersten Abschnitt (201) größer ist als der Abstand zwischen Ketten (210) in dem zweiten Abschnitt (200).

5. Faden nach einem der vorhergehenden Ansprüche, wobei die Schüsse (301) um zumindest einige (300) der Ketten in dem zweiten Abschnitt gewunden sind.

6. Faden nach einem der vorhergehenden Ansprüche, wobei sich die Dicke des Fadens zwischen den ersten und zweiten Abschnitten gemäß eines linearen oder gekurvten Profils verjüngt.

7. Faden nach einem der vorhergehenden Ansprüche, umfassend ein sich über einen Bereich (102) des Fadens erstreckendes flaches, im Wesentlichen ebenes Profil.

8. Faden nach einem der vorhergehenden Ansprüche, wobei sich die ersten und zweiten Abschnitte über im Wesentlichen die gesamte Länge des Fadens wiederholen.

9. Faden nach einem der Ansprüche 1 bis 7, wobei sich die ersten und zweiten Abschnitte über Endregionen (402) des Fadens erstrecken, und wobei ein Bereich (400) zwischen den Endregionen frei von den ersten und zweiten Abschnitten ist.

10. Faden nach Anspruch 9, wobei der Bereich zwischen den Endregionen eine im Wesentlichen gleichförmige Breite entlang seiner Länge aufweist.

11. Faden nach einem der vorhergehenden Ansprüche, wobei sich die Ketten über die gesamte Länge des Fadens erstrecken.

12. Faden nach einem der vorhergehenden Ansprüche, wobei sich die Ketten zwischen Endbereichen (106) und über einen zentralen Bereich (102) des Fadens erstrecken.

13. Verfahren zum Herstellen eines gewebten chirurgischen Fadens nach Anspruch 1, umfassend:
Verweben von Ketten (208, 210) und Schüssen (209, 211) zum Bilden eines sich über einen ersten Abschnitt (201) der Länge des Fadens erstreckenden ersten Webmusters (204);
Verweben von Ketten und Schüssen zum Bilden eines sich über einen zweiten Abschnitt (200) der Länge des Fadens erstreckenden zweiten Webmuster (203), wobei der zweite Abschnitt eine Fadenbreite (b) aufweist, die kleiner als eine Fadenbreite (a) des ersten Abschnitts ist;
Verweben der Ketten und Schüsse derart, dass ein Bereich (106) entlang einer Länge des Fadens eine Vielzahl von den ersten Abschnitten und eine Vielzahl von den zweiten Abschnitten aufweist, wobei sich die ersten und zweiten Abschnitte gemäß einer abwechselnden Anordnung über den Bereich des Fadens wiederholen, so dass der Faden entlang des Bereichs seiner Länge ein abwechselndes, durch Verbreiterungen (205) und Einschnürungen (206) geformtes Breitenprofil, welches sich transversal zu einer Längsachse des Fadens erstreckt, aufweist.

14. Verfahren nach Anspruch 13, umfassend das Verweben von Ketten (208) in dem ersten Abschnitt (201) mit einer größeren Spannung relativ zur der Spannung der Ketten (210) in dem zweiten Abschnitt (200).

15. Verfahren nach Anspruch 13, umfassend das Verweben von Schüssen (211) in dem zweiten Abschnitt (200) unter einer größeren Spannung relativ zu den Schüssen (209) in dem ersten Abschnitt (201).

## Revendications

1. Cordon chirurgical tissé comprenant :
des chaînes (208, 210) et des trames (209, 211) entrelacées s'étendant sur une longueur du cordon ;
un premier dessin d'armure (204) créé par les chaînes et les trames s'étendant sur une première section (201) de la longueur de cordon ;
un deuxième dessin d'armure (203) créé par les chaînes et les trames s'étendant sur une deuxième section (200) de la longueur du cordon, la deuxième section ayant une largeur de cordon (b) inférieure à une largeur de cordon (a) de la première section ;
**caractérisé en ce que**
une région (106) le long d'une longueur du cordon comprend une pluralité des premières sections et une pluralité des deuxièmes sections, les premières et deuxièmes sections se répétant selon un agencement alterné sur la région du cordon, l'agencement des premières et deuxièmes sections étant tel qu'il fournit un profil de largeur alterné formé à partir d'arêtes (205) et de creux (206) s'étendant transversalement par rapport à un axe longitudinal du cordon et le long de la région du cordon.

2. Cordon tel que revendiqué dans la revendication 1, dans lequel l'espacement entre des trames (209) dans la première section (201) est supérieur à l'espacement entre des trames (211) dans la deuxième section (200).

3. Cordon tel que revendiqué dans les revendications 1 et 2, comprenant un plus grand nombre de trames (211) dans la deuxième section (200) que dans la première section (201).

4. Cordon tel que revendiqué dans l'une des revendications précédentes, dans lequel l'espacement entre des chaînes (208) dans la première section (201) est supérieur à l'espacement entre des chaînes (210) dans la deuxième section (200).

5. Cordon tel que revendiqué dans l'une des revendications précédentes, dans lequel les trames (301) sont bouclées autour d'au moins certaines (300) des chaînes dans la deuxième section.

6. Cordon tel que revendiqué dans l'une des revendications précédentes, dans lequel l'épaisseur du cordon diminue entre les premières et deuxièmes sections selon un profil linéaire ou incurvé.

7. Cordon tel que revendiqué dans l'une des revendications précédentes, comprenant un profil plat, essentiellement planaire s'étendant sur une région (102) du cordon.

8. Cordon tel que revendiqué dans l'une des revendications précédentes, dans lequel les premières et deuxièmes sections se répètent sur essentiellement toute la longueur du cordon.

9. Cordon tel que revendiqué dans l'une quelconque des revendications 1 à 7, dans lequel les premières et deuxièmes sections s'étendent sur des régions d'extrémité (402) du cordon et où une région (400) entre les régions d'extrémité est dépourvue des premières et deuxièmes sections.

10. Cordon tel que revendiqué dans la revendication 9, dans lequel la région entre les régions d'extrémité comprend une largeur essentiellement uniforme le long de sa longueur.

11. Cordon tel que revendiqué dans l'une des revendications précédentes, dans lequel lesdites chaînes s'étendent sur toute la longueur dudit cordon.

12. Cordon tel que revendiqué dans l'une des revendications précédentes, dans lequel les chaînes s'étendent entre des régions d'extrémité (106) et à travers une région centrale (102) du cordon.

13. Procédé de fabrication d'un cordon chirurgical tissé selon la revendication 1, comprenant le fait :
d'entrelacer des chaînes (208, 210) et des trames (209, 211) pour créer un premier dessin d'armure (204) s'étendant sur une première section (201) d'une longueur du cordon ;
d'entrelacer des chaînes et des trames pour créer un deuxième dessin d'armure (203) s'étendant sur une deuxième section (200) de la longueur du cordon, la deuxième section ayant une largeur de cordon (b) inférieure à une largeur (a) de la première section ;
d'entrelacer les chaînes et les trames de sorte qu'une région (106) le long d'une longueur du cordon comprenne une pluralité des premières sections et une pluralité des deuxièmes sections, les premières et deuxièmes sections se répétant selon un agencement alterné sur la région du cordon de sorte que le cordon comprenne un profil de largeur alterné le long de la région de sa longueur formé à partir d'arêtes (205) et de creux (206) s'étendant transversalement par rapport à un axe longitudinal du cordon.

14. Procédé tel que revendiqué dans la revendication 13 comprenant le fait d'entrelacer des chaînes (208) dans la première section (201) à une tension plus élevée par rapport à la tension des chaînes (210) dans la deuxième section (200).

15. Procédé tel que revendiqué dans la revendication 13, comprenant le fait d'entrelacer des trames (211) sous une tension plus élevée dans la deuxième section (200) par rapport à celle des trames (209) dans la première section (201).
